# EUROPEAN PATENT APPLICATION

(11) **EP 4 491 773 A1**
(43) Date of publication of application: **15.01.2025**
(21) Application number: 24181205.6
(22) Date of filing: 10.06.2024
(51) Int. Cl.: C25D 11/02, C25D 11/04, C25D 11/26, C25D 11/30, C25D 11/34, A61L 2/232

(54) **ANTIMICROBIAL OXIDIZED SURFACE**

(30) Priority: 13.06.2023 US 202363472623 P
(71) Applicant: Vapor Technologies, Inc., Longmont CO 80503 (US)
(72) Inventor: ANTON, Bryce Randolph, Longmont, 80503 (US); KUNRATH, Augusto, Denver, 80220 (US)
(74) Representative: Keltie LLP

(57) **Abstract**

A method for forming an article coated with an antimicrobial coating includes a step of depositing a mixed metal layer over a substrate. Characteristically, the mixed metal layer includes copper and an oxidizable metal. The mixed metal layer is oxidized in an electrolyte solution by anodization or by applying a sufficient voltage to a surface of the mixed metal layer sufficient to form an electrical discharge that oxidizes at least a portion of the mixed metal layer to form an antimicrobial coppercontaining layer that includes a mixture of oxides of copper and the oxidizable metal.

## Description

### CROSS-REFERENCE TO RELATED APPLICATIONS

This application claims the benefit of U.S. provisional application Serial No. 63/472,623 filed June 13, 2023, the disclosure of which is hereby incorporated in its entirety by reference herein.

### TECHNICAL FIELD

In at least one aspect, the present invention is related to antimicrobial coatings, and to articles coated with such coatings.

### BACKGROUND

Coatings with bioactive properties can be useful for various purposes. Bioactive refers to a material having biological effects or physiological effects on living things. For example, a bioactive material includes a material resulting in a modification in the normal biological function or a physiological mechanism of a living thing. Bioactive, as used herein, includes beneficial and detrimental effects to microorganisms or modifications to the normal functioning of a microorganism. One common bioactive material is antimicrobial substances. Antimicrobial coatings serve many purposes. Generally, antimicrobial coatings inhibit the growth or kill microbes like viral, bacterial or fungal organisms. One particularly relevant example includes preventing the spread of communicable diseases by the use of antimicrobial materials. Antimicrobial materials may also serve hygienic purposes. The desire to prevent the spread of disease and for heightened hygiene has resulted in significant efforts to develop antimicrobial materials. The use of antimicrobial materials in health care facilities and health treatments can provide significant benefits. Health facilities, such as hospitals, present unique environments that combine high concentrations of germs and individuals with vulnerable immunities. Therefore, facilities such as hospitals greatly benefit from antimicrobial surfaces. Antimicrobial materials for medical equipment can reduce the burden of disinfecting and prevent the spread of disease. Further, affordable antimicrobial surfaces could present benefits on any surface that comes into contact with living things. For example, surfaces involved in cooking or commonly touched surfaces like doorknobs could greatly benefit from antimicrobial properties. Even primarily decorative surfaces, if affordable, could benefit from antimicrobial characteristics and assist in inhibiting the spread and growth of harmful or undesirable antimicrobial life.

Producing antimicrobial materials can be difficult and expensive. Further, antimicrobial properties may have other undesirable properties. For example, some antimicrobial materials may be too soft. Other antimicrobial materials may have poor abrasion resistance. Microban^{®} is an antimicrobial coating that includes silver particles dispersed in an organic matrix. Antimicrobial coatings involving an organic matrix may have the aesthetic appearance of paint. In some applications, the appearance of paint may be undesirable. Some antimicrobial coatings may use nanoparticle vapor deposition to deposit nanoparticles with antimicrobial properties on the surface of a coating. For example, ABACO^{®} from Protec, is an antimicrobial coating using nanoparticles. However, the use of nanoparticles can be complex and expensive. Further such coatings may have delicate surfaces or poor abrasion resistance. Another example of antimicrobial materials includes various metals. For example, silver is known to have antimicrobial properties. However, as stated above silver can be expensive, and its properties may not be suitable for many applications. For example, the appearance or abrasion resistance of silver may be unsuitable for certain applications.

Accordingly, there is a need for an antimicrobial coating that solves one or more of these problems or offers an alternative to current antimicrobial materials.

### SUMMARY

In at least one aspect, a method for forming an article coated with an antimicrobial coating is provided. The method includes a step of depositing a mixed metal layer over a substrate. Characteristically, the mixed metal layer includes copper and an oxidizable metal. The mixed metal layer is oxidized in an electrolyte solution by anodization or by applying a sufficient voltage to a surface of the mixed metal layer sufficient to form an electrical discharge that oxidizes at least a portion of the mixed metal layer to form an antimicrobial copper-containing layer that includes a mixture of oxides of copper and the oxidizable metal.

In another aspect, a coated article is provided. The coated article includes a substrate composed of an oxidizable metal, a metal oxide layer disposed over the substrate, and an antimicrobial copper-containing layer disposed over the metal oxide layer. Characteristically, the metal oxide layer being composed of the oxidizable metal. Advantageously, the antimicrobial copper-containing layer including a mixture of oxides of copper and the oxidizable metal.

In another aspect, a coated article is provided. The coated article includes a substrate composed of a metal or metal alloy, a mixed metal layer disposed over the substrate, and an antimicrobial copper-containing layer disposed over the mixed metal layer. Characteristically, the mixed metal layer includes copper and an oxidizable metal. Advantageously, the antimicrobial copper-containing layer includes a mixture of oxides of copper and the oxidizable metal.

The foregoing summary is illustrative only and is not intended to be in any way limiting. In addition to the illustrative aspects, embodiments, and features described above, further aspects, embodiments, and features will become apparent by reference to the drawings and the following detailed description.

### BRIEF DESCRIPTION OF THE DRAWINGS

For a further understanding of the nature, objects, and advantages of the present disclosure, reference should be had to the following detailed description, read in conjunction with the following drawings, wherein like reference numerals denote like elements and wherein:
FIGURE 1A. Flowchart depicting a method for forming an article coated with an antimicrobial coating.
FIGURE 1B. Flowchart depicting a method for forming an article coated with an antimicrobial coating.
FIGURE 2A. Schematic of a coated article.
FIGURE 2B. Schematic of a coated article.

### DETAILED DESCRIPTION

Reference will now be made in detail to presently preferred compositions, embodiments and methods of the present invention, which constitute the best modes of practicing the invention presently known to the inventors. The Figures are not necessarily to scale. However, it is to be understood that the disclosed embodiments are merely exemplary of the invention that may be embodied in various and alternative forms. Therefore, specific details disclosed herein are not to be interpreted as limiting, but merely as a representative basis for any aspect of the invention and/or as a representative basis for teaching one skilled in the art to variously employ the present invention.

Except in the examples, or where otherwise expressly indicated, all numerical quantities in this description indicating amounts of material or conditions of reaction and/or use are to be understood as modified by the word "about" in describing the broadest scope of the invention. Practice within the numerical limits stated is generally preferred. Also, unless expressly stated to the contrary: percent, "parts of," and ratio values are by weight; molecular weights provided for any polymers refers to weight average molecular weight unless otherwise indicated; the description of a group or class of materials as suitable or preferred for a given purpose in connection with the invention implies that mixtures of any two or more of the members of the group or class are equally suitable or preferred; description of constituents in chemical terms refers to the constituents at the time of addition to any combination specified in the description, and does not necessarily preclude chemical interactions among the constituents of a mixture once mixed; the first definition of an acronym or other abbreviation applies to all subsequent uses herein of the same abbreviation and applies mutatis mutandis to normal grammatical variations of the initially defined abbreviation; and, unless expressly stated to the contrary, measurement of a property is determined by the same technique as previously or later referenced for the same property.

It is also to be understood that this invention is not limited to the specific embodiments and methods described below, as specific components and/or conditions may, of course, vary. Furthermore, the terminology used herein is used only for the purpose of describing particular embodiments of the present invention and is not intended to be limiting in any way.

It must also be noted that, as used in the specification and the appended claims, the singular form "a," "an," and "the" comprise plural referents unless the context clearly indicates otherwise. For example, reference to a component in the singular is intended to comprise a plurality of components.

The term "comprising" is synonymous with "including," "having," "containing," or "characterized by." These terms are inclusive and open-ended and do not exclude additional, unrecited elements or method steps.

The phrase "consisting of" excludes any element, step, or ingredient not specified in the claim. When this phrase appears in a clause of the body of a claim, rather than immediately following the preamble, it limits only the element set forth in that clause; other elements are not excluded from the claim as a whole.

The phrase "consisting essentially of" limits the scope of a claim to the specified materials or steps, plus those that do not materially affect the basic and novel characteristic(s) of the claimed subject matter.

With respect to the terms "comprising," "consisting of," and "consisting essentially of," where one of these three terms is used herein, the presently disclosed and claimed subject matter can include the use of either of the other two terms.

The phrase "composed of" means "including" or "comprising." Typically, this phrase is used to denote that an object is formed from a material.

It should also be appreciated that integer ranges explicitly include all intervening integers. For example, the integer range 1-10 explicitly includes 1, 2, 3, 4, 5, 6, 7, 8, 9, and 10. Similarly, the range 1 to 100 includes 1, 2, 3, 4.... 97, 98, 99, 100. Similarly, when any range is called for, intervening numbers that are increments of the difference between the upper limit and the lower limit divided by 10 can be taken as alternative upper or lower limits. For example, if the range is 1.1. to 2.1 the following numbers 1.2, 1.3, 1.4, 1.5, 1.6, 1.7, 1.8, 1.9, and 2.0 can be selected as lower or upper limits. In the specific examples set forth herein, concentrations, temperature, and reaction conditions (e.g. pressure, pH, etc.) can be practiced with plus or minus 50 percent of the values indicated rounded to three significant figures. In a refinement, concentrations, temperature, and reaction conditions (e.g., pressure, pH, etc.) can be practiced with plus or minus 30 percent of the values indicated rounded to three significant figures of the value provided in the examples. In another refinement, concentrations, temperature, and reaction conditions (e.g., pH, etc.) can be practiced with plus or minus 10 percent of the values indicated rounded to three significant figures of the value provided in the examples.

The term "a copper oxide" means a compound having formula CuₓO where x is from 2.2 to 0.6. In a refinement, z is at least 0.6, 0.7, 0.9, or 0.95 and at most 2.2, 2.0, 1.8, 1.6, 1.4 or 1.2.

The term "a zirconium oxide" means a compound having formula ZrO_{y} where y is from 1.7 to 2.3. An example of a zirconium oxide is ZrO₂.

The term "a titanium oxide" means a compound having formula TiO_{z} where z is from 1.7 to 2.3. In a refinement, a titanium oxide means a compound having formula TiO_{z} where z is from 1.7 to 1.9. In another refinement, a titanium oxide means a compound having formula TiO_{z} where z is from 1.8 to 2.0. In another refinement, a titanium oxide means a compound having formula TiO_{z} where z is from 1.9 to 2.1. In another refinement, a titanium oxide means a compound having formula TiOz where z is from 2.0 to 2.2. In another refinement, a titanium oxide means a compound having formula TiO_{z} where z is from 2.1 to 2.3. In another refinement, a titanium oxide means a compound having formula TiO_{z} where z is from 1.7 to 2.0. In another refinement, a titanium oxide means a compound having formula TiO_{z} where z is from 1.8 to 2.1. In another refinement, a titanium oxide means a compound having formula TiO_{z} where z is from 1.9 to 2.2. In another refinement, a titanium oxide means a compound having formula TiO_{z} where z is from 2.0 to 2.3. In another refinement, a titanium oxide means a compound having formula TiO_{z} where z is from 1.7 to 2.1. In another refinement, a titanium oxide means a compound having formula TiO_{z} where z is from 1.8 to 2.2. In another refinement, a titanium oxide means a compound having formula TiO_{z} where z is from 1.9 to 2.3. An example of a titanium oxide is TiO₂.

The term "an aluminum oxide" means a compound having formula Al₂O_{w} where w is from 2.7 to 3.3. In a refinement, an aluminum oxide means a compound Al₂O_{w} where w is from 2.7 to 2.9. In another refinement, an aluminum oxide means a compound Al₂O_{w} where w is from 2.8 to 3.0. In another refinement, an aluminum oxide means a compound Al₂O_{w} where w is from 2.9 to 3.1. In another refinement, an aluminum oxide means a compound Al₂O_{w} where w is from 3.0 to 3.2. In another refinement, an aluminum oxide means a compound Al₂O_{w} where w is from 3.1 to 3.3. In another refinement, an aluminum oxide means a compound Al₂O_{w} where w is from 2.7 to 3.0. In another refinement, an aluminum oxide means a compound Al₂O_{w} where w is from 2.8 to 3.1. In another refinement, an aluminum oxide means a compound Al₂O_{w} where w is from 2.9 to 3.2. In another refinement, an aluminum oxide means a compound Al₂O_{w} where w is from 3.0 to 3.3. In another refinement, an aluminum oxide means a compound Al₂O_{w} where w is from 2.7 to 3.1. In another refinement, an aluminum oxide means a compound Al₂O_{w} where w is from 2.8 to 3.2. In another refinement, an aluminum oxide means a compound Al₂O_{w} where w is from 2.9 to 3.3. An example of an aluminum oxide is Al₂O₃.

The term "a niobium oxide" means a compound having formula NO_{w} where w is from 0.8 to 2.3. In a refinement, a niobium oxide means a compound having formula NO_{w} where w is from 0.8 to 1.0. In another refinement, a niobium oxide means a compound having formula NO_{w} where w is from 0.9 to 1.1. In another refinement, a niobium oxide means a compound having formula NO_{w} where w is from 1.0 to 1.2. In another refinement, a niobium oxide means a compound having formula NO_{w} where w is from 1.1 to 1.3. In another refinement, a niobium oxide means a compound having formula NO_{w} where w is from 1.2 to 1.4. In another refinement, a niobium oxide means a compound having formula NO_{w} where w is from 1.3 to 1.5. In another refinement, a niobium oxide means a compound having formula NO_{w} where w is from 1.4 to 1.6. In another refinement, a niobium oxide means a compound having formula NO_{w} where w is from 1.5 to 1.7. In another refinement, a niobium oxide means a compound having formula NO_{w} where w is from 1.6 to 1.8. In another refinement, a niobium oxide means a compound having formula NO_{w} where w is from 1.7 to 1.9. In another refinement, a niobium oxide means a compound having formula NO_{w} where w is from 1.8 to 2.0. In another refinement, a niobium oxide means a compound having formula NO_{w} where w is from 1.9 to 2.1. In another refinement, a niobium oxide means a compound having formula NO_{w} where w is from 2.0 to 2.2. In another refinement, a niobium oxide means a compound having formula NO_{w} where w is from 2.1 to 2.3. Examples of niobium oxide are NbO, NbO₂, and NbO₂.

The term "standard electrode potential" means the electrical potential (i.e., the voltage developed) of a reversible electrode at standard state in which solutes are at an effective concentration of 1 mol/liter, the activity for each pure solid, pure liquid, or for water (solvent) is 1, the pressure of each gaseous reagent is 1 atm., and the temperature is 25° C. Standard electrode potentials are reduction potentials. Standard electrode potential is the voltage difference between a half-cell and a standard hydrogen electrode (SHE) at standard conditions of temperature, pressure, and ion concentration (e.g., 25°C, 1 atm, and 1 M concentration).

The term "antimicrobial" means a material or material surface that kills or inhibits the growth of bacteria, viruses, and/or fungi.

### Abbreviations:

"MAO" means micro-arc oxidation.
"PEO" means plasma electrolytic oxidation.

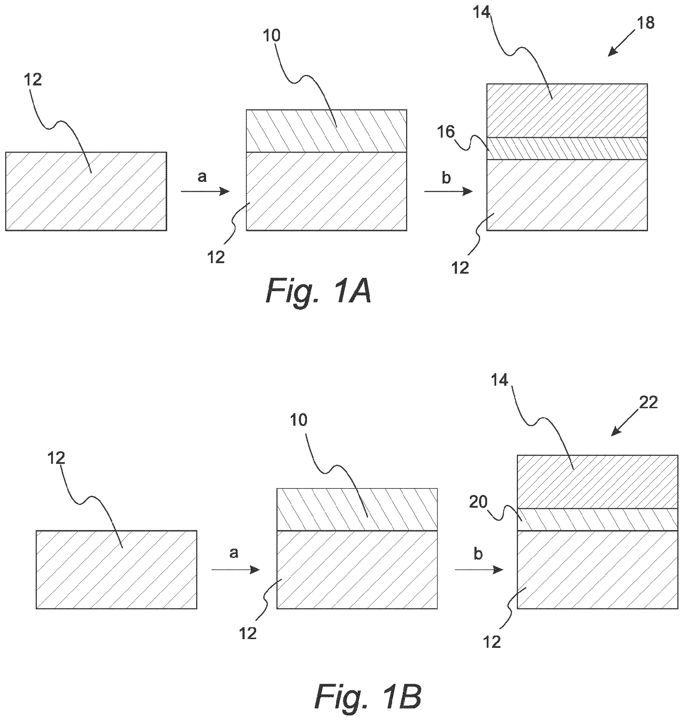

Referring to Figures 1A and 1B, flowcharts depicting a method for forming an article coated with an antimicrobial coating are provided. In step a) mixed metal layer 10 is deposited over a substrate 12. The mixed metal layer 10 includes an oxidizable metal. Typically, mixed metal layer 10 includes copper and an oxidizable metal. In step b), mixed metal layer 10 is oxidized in an electrolyte solution by applying a sufficient voltage to a surface of the mixed metal layer to form an electrical discharge that oxidizes at least a portion of the mixed metal layer to form an antimicrobial copper-containing layer 14 that includes a mixture of oxides of copper and the oxidizable metal. In a refinement, mixed metal layer 10 is substantially completely oxidized. The voltage applied can vary depending on the specific requirements of the process. For example, the oxides of the oxidizable metal can be a niobium oxide, a zirconium oxide, an aluminum oxide, and/or a titanium oxide. The oxide of copper can be a copper oxide. In an aspect, the antimicrobial copper-containing layer 14 can be viewed as dispersed in a metal oxide matrix of the oxide of oxidizable metal. This provides better control over the amount and would be expected to have superior durability. Additionally, would be able to apply to a variety of substrate materials.

Figure 1A depicts a variation in which the mixed metal layer 10 is oxidized for a sufficient time that a portion of the substrate is oxidized to metal oxide layer 16 to form coated article 18. In a refinement, mixed metal layer 10 is substantially completely oxidized. In a refinement, the substrate is composed of the oxidizable metal or an alloy including the oxidizable metal. In a further refinement, the oxidizable metal is Mg, Ti, Al, Zr, or Nb.

Figure 1B depicts a variation in which mixed metal layer 10 is oxidized for a sufficient time that a portion of the mixed metal layer remains unoxidized as mixed metal interlayer 20 to form coated article 22. In a refinement, the substrate is composed of an oxidation-resistant metal or a metal alloy. Examples of oxidation-resistant metal for metal alloys include stainless steel. In another refinement, the substrate is composed of the oxidizable metal or an alloy including the oxidizable metal. In a further refinement, the oxidizable metal is Mg, Ti, Al, Zr, or Nb. In another refinement, the substrate is chrome-plated.

In one variation, the mixed metal layer is oxidized by anodization. In the anodization process, the metal substrate is contacted with an electrolyte solution that includes oxygen ions (e.g., an aqueous solution). The electrolytic solution typically includes an oxidizing agent (e.g., sulfuric acid or chromic acid) to facilitate the formation of the oxide layer. The positive terminal of a DC power source is connected to a metal substrate to form an oxide layer.

In another aspect, the mixed metal layer is oxidized by plasma electrolytic oxidation. In the plasma electrolytic oxidation process the metal substrate is contacted with an electrolyte solution that includes oxygen ions (e.g., an aqueous solution). A high voltage is continuously applied to the metal surface at a sufficient voltage to cause a plasma discharge to form. The high voltage is typically greater than 200 V. A useful range for the high voltage is from 300 V to 800 V.

In another aspect, the mixed metal layer is oxidized by micro-arc oxidation. In the micro-arc oxidation process, the metal substrate is contacted with an electrolyte solution that includes oxygen ions (e.g., an aqueous solution). A high voltage is applied to the metal surface at a sufficient voltage to cause a series of micro-discharges to form. Micro-arc oxidation is an electrochemical surface treatment process used to create oxide coatings on metals such as aluminum, magnesium, titanium, and their alloys. This process can enhance the surface properties of the metal, such as hardness, wear resistance, and corrosion resistance. In Micro-arc oxidation, a metal workpiece acts as the anode and is submerged in an electrolyte solution. In a refinement, the electrolyte can include alkaline substances like potassium hydroxide (KOH) or sodium silicate (Na₂SiO₃). A high voltage (several hundred to several thousand volts) is applied between the anode (workpiece) and the cathode (counter electrode), which is significantly higher than the voltages used in conventional anodizing processes. For example, the high voltage is typically greater than 1000 V. A useful range for the high voltage is from 10 kV to 30 kV. In micro-arc oxidation, the voltage can be applied in short high-intensity bursts. At high voltage, dielectric breakdown of the oxide layer occurs, leading to the formation of micro-discharges or plasma arcs on the surface of the metal. These micro-discharges create localized high temperatures and pressures. The high-energy micro-discharges cause the metal to react with the electrolyte, forming a thick, dense oxide layer. The oxide layer grows both inward and outward from the original metal surface.

As set forth above, mixed metal layer 10 can be composed of an oxidizable metal. Similarly, the substrate can be composed of an oxidizable metal. In a refinement, the oxidizable metal has a standard electrode potential less than 0 V. In some refinements, the oxidizable metal has a standard electrode potential less than 0 V, -0.2 V, -0.5 V, -0.8 V, -1.0 V, or -1.2 V. Examples of the oxidizable metal include Mg, Ti, Al, Zr, or Nb.

In a variation, antimicrobial copper-containing layer includes a copper oxide. In a refinement, antimicrobial copper-containing layer includes a compound having formula Cu_{z}O where z is from 2.2 to 0.8. In a refinement, the antimicrobial copper-containing layer includes cupric oxide (i.e., CuO). In further refinement, the antimicrobial copper-containing layer includes cuprous oxide (i.e., Cu₂O).

In a variation, the antimicrobial copper-containing layer has a thickness greater than about 0.5 microns. In a refinement, antimicrobial copper-containing layer has a thickness from about 0.5 microns to about 2 microns.

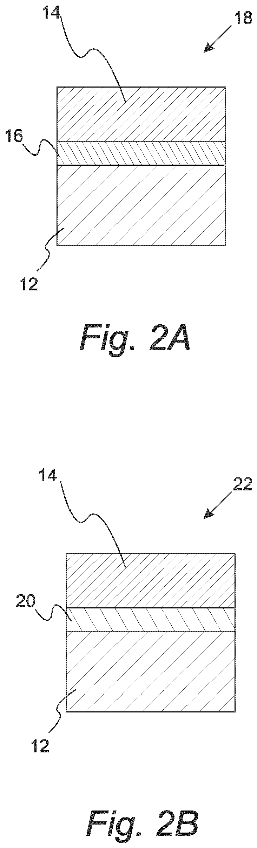

Referring to Figure 2A, a schematic of a coated article is provided. Coated article 18 includes a substrate 12 composed of an oxidizable metal, a metal oxide layer 16 disposed over and optionally contacting the substrate, and an antimicrobial copper-containing layer 14 disposed over and optionally contacting the metal oxide layer. Characteristically, the metal oxide layer is composed of an oxidizable metal. Advantageously, the antimicrobial copper-containing layer includes a mixture of oxides of copper and the oxidizable metal.

Referring to Figure 2B, a schematic of a coated article is provided. Coated article 22 includes a substrate 12 composed of a metal or metal alloy, a mixed metal layer 20 disposed over the substrate 12, and an antimicrobial copper-containing layer 14 disposed over the mixed metal layer. Characteristically, the mixed metal layer includes copper and an oxidizable metal. Characteristically, the mixed metal layer includes copper and an oxidizable metal. Advantageously, the antimicrobial copper-containing layer includes a mixture of oxides of copper and the oxidizable metal.

While exemplary embodiments are described above, it is not intended that these embodiments describe all possible forms of the invention. Rather, the words used in the specification are words of description rather than limitation, and it is understood that various changes may be made without departing from the spirit and scope of the invention. Characteristically, the mixed metal layer includes copper and an oxidizable metal. Advantageously, the antimicrobial copper-containing layer includes a mixture of oxides of copper and the oxidizable metal.

## Claims

1. A method for forming an article coated with an antimicrobial coating, the method comprising:
depositing a mixed metal layer over a substrate, the mixed metal layer including copper and an oxidizable metal; and
oxidizing the mixed metal layer in an electrolyte solution by anodization or by applying a sufficient voltage to a surface of the mixed metal layer sufficient to form an electrical discharge that oxidizes at least a portion of the mixed metal layer to form an antimicrobial copper-containing layer that includes a mixture of oxides of copper and the oxidizable metal.

2. The method of claim 1, wherein the mixed metal layer is oxidized for a sufficient time that a portion of the mixed metal layer remains unoxidized.

3. The method of claims 1 or 2, wherein the substrate is composed of the oxidizable metal or an alloy including the oxidizable metal, and the oxidizable metal is Mg, Ti, Al, Zr, or Nb.

4. The method of any one of the preceding claims, wherein the substrate is composed of an oxidation-resistant metal or a metal alloy.

5. The method of any one of the preceding claims, wherein the substrate is chrome plated.

6. The method of any one of the preceding claims, wherein the mixed metal layer is oxidized by anodization, micro-arc oxidation, or plasma electrolytic oxidation.

7. The method of any one of the preceding claims, wherein the oxidizable metal has a standard electrode potential less than 0 V.

8. The method of any one of the preceding claims, wherein the antimicrobial copper-containing layer includes a compound having formula Cu_{z}O where z is from 2.2 to 0.6, cupric oxide, or cuprous oxide.

9. The method of any one of the preceding claims, wherein the antimicrobial copper-containing layer has a thickness from about 0.5 microns to about 2 microns.

10. A coated article comprising:
a substrate composed of an oxidizable metal;
a metal oxide layer disposed over the substrate, the metal oxide layer being composed of the oxidizable metal; and
an antimicrobial copper-containing layer disposed over the metal oxide layer, the antimicrobial copper-containing layer including a mixture of oxides of copper and the oxidizable metal.

11. The coated article of claim 10, wherein the oxidizable metal has a standard electrode potential less than 0 V.

12. The coated article of claim 10 or claim 11, wherein the oxidizable metal is Ti, Al, Zr, or Nb.

13. The coated article of any one of claims 10 to 12, wherein the antimicrobial copper-containing layer includes a compound having formula Cu_{z}O where z is from 2.2 to 0.6, cupric oxide, or cuprous oxide.

14. The coated article of any one of claims 10 to 13, wherein the antimicrobial copper-containing layer has a thickness from about 0.5 microns to about 2 microns.

15. A coated article comprising:
a substrate composed of a metal or metal alloy;
a mixed metal layer disposed over the substrate, the mixed metal layer including copper and an oxidizable metal; and
an antimicrobial copper-containing layer disposed over the mixed metal layer, the antimicrobial copper-containing layer including a mixture of oxides of copper and the oxidizable metal.
